Europäisches Patentamt

European Patent Office

Office européen des brevets

(11) Publication number: 0 294 979
A1

# EUROPEAN PATENT APPLICATION

(21) Application number: 88304906.6

(22) Date of filing: 31.05.88

(51) Int. Cl.4: **C12N 15/00 , C07H 21/04 , C12N 1/20 , C12P 21/00**

(30) Priority: 10.06.87 GB 8713559

(43) Date of publication of application:
**14.12.88 Bulletin 88/50**

(84) Designated Contracting States:
**AT BE CH DE ES FR GB GR IT LI LU NL SE**

(71) Applicant: **PA Consulting Services Limited**
**Hyde Park House 60a Knightsbridge**
**London SW1X 7LE(GB)**

(72) Inventor: **Edwards, Richard Mark**
**36 Chinnor Road Thame**
**Oxford Oxfordshire(GB)**
Inventor: **Light, Janice Ann**
**12, Granby Road**
**Old Stevenage Hertfordshire(GB)**
Inventor: **Nicholson, Kim**
**101 High Street Melbourn**
**Royston Hertfordshire(GB)**

(74) Representative: **Matthews, Heather Clare et al**
**Keith W Nash & Co Pearl Assurance House**
**90-92 Regent Street**
**Cambridge CB2 1DP(GB)**

(54) Improvements in or relating to structural proteins.

(57) A recombinant plasmid contains DNA coding for the amino acid sequence (C):
(GAGAGSGAAG(GAGAGS)$_8$ Y)

The DNA preferably codes for protein of the following form:
met(C)$_n$A1(C)$_n$A1(C)$_n$A1(C)$_n$A1A2(TerTerTer)

where al and A2 are amorphous regions separating groups of units of crystalline structure. Such a protein may have properties similar to those of B mori fibroin.

EP 0 294 979 A1

## Improvements in or relating to structural proteins

Field of the Invention

This invention relates to structural proteins, particularly silk-like proteins.

Background to the Invention

Silk is a protein fibre produced by spinning which is produced naturally by the larvae of the Bombyx mori moth and many other Arthropods. The Bombyx mori moth larva synthesises the components of silk inside its body and extrudes them in the form of a fibre of silk which it spins into a cocoon. The B. mori silk fibre consists of twin filaments of the protein fibroin which are cemented or glued together by the protein sericin. These moth cocoons are the basis of commercial natural silk production; the larvae produce fibres with a composition and structure that result in natural silk having a unique combination of properties of strength, softness and lustre, which cannot be matched by conventional manmade fibres.

Production of natural silk from these moth larvae, however, is limited both in quantity and quality by geographical, political and other factors. As a result, the supply of natural silk is limited and prices are high.

It is an object of the present invention to facilitate production of silk-like structural proteins, based on the protein fibroin of the silk fibre produced by the larvae of the Bombyx mori moth, by a method which does not involve the Bombyx mori moth larvae, or other Arthropods.

It is also an object of the invention to facilitate production of components of novel silk-like structural proteins having specific properties not necessarily found in naturally produced silks.

The Invention

According to one aspect of the invention there is provided a recombinant plasmid containing DNA coding for the amino acid sequence:

(GAGAGSGAAG(GAGAGS)$_8$Y)

In the specified sequence of 59 amino acids (which will be referred to as the 59 mer unit or crystalline unit repeat C), the conventional symbols are used for the constituent amino acids, namely:

A = alanine
G = glycine
S = serine
Y = tyrosine

The plasmid may contain DNA coding for several of the 59 mer units, the units being for example arranged in one or more (eg 2 to 6) groups each comprising a number, eg 4 to 8, of contiguous units. Such groups are of closely packed or crystalline structure, and may be separated by smaller regions which are less well organised, ie of amorphous structure. Such amorphous regions or groups are preferably based on a small portion of the enzyme betagalactosidase. This arrangement is convenient, as the gene for the crystalline groups may be fused to the betagalactosidase gene which acts as a reporter for expression.

A preferred amorphous group (called A1) is based on part of the lac Z gene and has the following amino acid sequence:

(KNSLAVVLQRRDWENPGVTQLNRLAAHPPFASTM)

As before, the conventional symbols are used for the constituent amino acids, namely:

C = Cysteine
D = Aspartic acid
E = Glutamic acid
F = Phenylalanine
H = Histidine
K = Lysine
L = Leucine
M = Methionine
N = Asparagine
P = Proline

Q = Glutamine
R = Arginine
T = Threonine
V = Valine
W = Tryptophan

The amino acid sequence preferably includes a different amorphous group at one end. One preferred group for this purpose (called A2) is the amino acid sequence at the end of lac Z gene remaining after Pvu II cleavage and (using the conventional symbols) is as follows:
(VSLAGRYHYQLVWCQK)

Thus, protein for which the DNA codes may be of the following form:
met(C)$_n$A1(C)$_n$A1(C)$_n$A1(C)$_n$A1A2 (TerTerTer)

If the number of crystalline unit repeats in each crystalline group is 4 ( ie n = 4), the protein has a total size of 1101 amino acids and is encoded by a 3.3kb gene.

The plasmid may be produced by inserting appropriate genetic material into a suitable plasmid vector. One preferred vector is derived from the plasmid pATI53.

The invention also concerns DNA coding for the 59 mer unit.

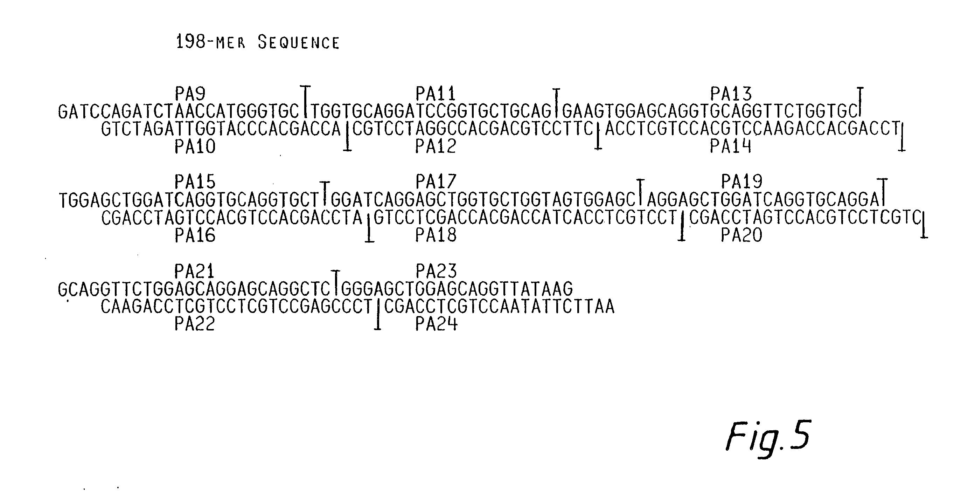

In a further aspect, the present invention provides DNA consisting of the base sequence shown in Figure 5.

The invention also provides a recombinant plasmid containing DNA consisting of the basic sequence shown in Figure 5.

The plasmid may be introduced into a suitable microbial host for cloning and expression of protein containing the 59 mer.

Hence, in a further aspect the present invention provides a microorganism into which a plasmid embodying the invention has been introduced.

Examples of possible host microorganisims include:

Escherichia coli
Saccharomyces cerevisiae
Pseudomonas spp
Rhodopseudomonas spp
Bacillus subtilis and other Bacilli
Streptomyces coelicolor and other Streptomycetes
Methylomonas spp
E. coli is the presently preferred host microorganism.

The invention also provides a method of producing protein including the 59 mer unit, comprising inserting a plasmid in accordance with the invention into a suitable microbial host in which the protein is expressed.

The protein product may be produced by an appropriate microorganism (and extracted from the microorganism cells if produced intracellularly) by appropriate treatment, eg by a fermentation process.

The invention also includes within its scope protein produced by the method of the invention.

The protein may have properties similar to those of B. mori fibroin because of similarities in structure. B. mori fibroin consists of approximately 10 domains of closely packed or crystalline material in which the 59 mer unit occurs, interspersed with less well organised or amorphous regions which are more variable in their amino acid content but generally contain more amino acids with bulky side groups, resulting in a less ordered structure in these regions. Thus, there are similarities with embodiments of the invention in which the protein comprises groups of contiguous 59 mer units interspersed with amorphous regions. Crystalline groups consisting of 4 to 8 of the 59 mer units are similar in size to a single crystalline domain in B. mori fibroin. As compared with B. mori fibroin, such proteins have crystalline structures of greater uniformity: variations found in natural proteins are removed. Also, the amorphous group A1 discussed above is smaller than that found in B. mori fibroin and has a very different sequence composition.

By modifying the plasmid, so the properties of the resulting protein may be varied as required. For example, inclusion of different amorphous regions, interspersed with the crystalline regions, can confer a range of physical properties, such as a range of extensibilities, on a final fibrous product. Properties may also be varied by varying the length and sequence composition of the amorphous regions and crystalline regions: this can be effected by synthesising new nucleotide sequence fragments and replacing parts of the original sequences using restriction endonuclease sites designed for this purpose. By such variations, materials having specified properties suited to particular requirements and applications may be produced.

In appropriate cases, the protein product may be treated and spun to produce a silk-like fibre. Such a fibre may have properties similar to or indistinguishable from those of natural silk, or may have deliberately modified properties as noted above. Thus, the invention can be used for the production of modified or designed silks having specified properties, not necessarily found in nature, eg designed for particular industrial materials applications.

The invention will be further described in the following illustrative Example which refers to the accompanying drawings in which:

Figure 1 illustrates the base sequence of a synthetic transcription terminator;

Figure 2 illustrates plasmid pPA10;

Figure 3 illustrates the EcoRI-SalI fragment of plasmid PPA1;

Figure 4 illustrates plasmid pPA2;

Figure 5 illustrates the base sequence of a 198bp gene;

Figure 6 illustrates plasmid pPA5;

Figure 7 illustrates the sequence of individual oligonucleotides comprising the 198bp gene of Figure 5;

Figure 8 illustrates double stranded oligonucleotides formed during production of the 198bp gene of Figure 5;

Figure 9 illustrates further oligonucleotides formed during producion of the 198bp gene of Figure 5;

Figure 10 illustrates diagramatically the 198bp fragment;

Figure 11 illustrates a 177bp extension of the gene of Figure 5;

Figure 12 illustrates plasmid pPA6;

Figure 13 illustrates plasmid pPA7;

Figure 14 illustrates plasmid pPA8;

Figure 15 illustrates plasmid pPA9;

Figure 16 illustrates a 10bp Ncol linker; and

Figure 17 illustrates restriction sites of plasmid pPA9.

## Description of Preferred Embodiment

## Example

The following example concerns a silk-like protein based on the 59 amino acid unit.

## General

The microbial protein of interest consists of 2 to 6 crystalline regions each containing the 59 mer unit repeated contiguously 4 to 8 times, the crystalline regions being separated by smaller "amorphous" regions. The 59 amino acid sequence is based on a sequence found in natural silk, in the protein fibroin produced by B. mori larvae. The crystalline structure of the microbial protein is totally uniform: variations found in natural proteins are removed. The amorphous region is smaller than that found in natural B. mori silks and has a very different sequence composition: the example is based on part of the lac Z gene for convenience.

The combination of regularity of crystalline structure and shortness of amorphous region provides a molecule which, although based on B. mori silk, is likely to behave differently, and can be used as a model system to be adapted according to specific material needs.

## Necessary elements, for expression of a gene in a bacterial host (E. coli in the Example).

Elements                                Chosen

Vector                                  pPAl (a derivative of pAT153-
                                        see gene assembly section)

Promotor                                tac

Ribosome binding site to                AGGATCTAACCATG etc.
initiator codon

Silk gene                               see below

Stop codon                              (lac Z)TAATAATAA

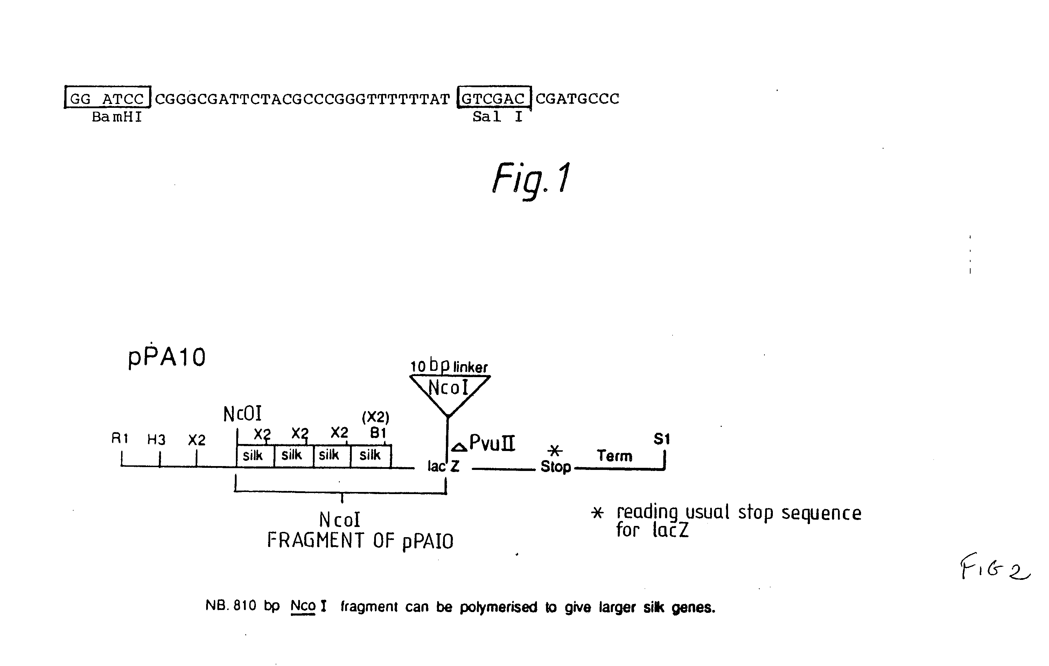

Terminator                              A synthetic transcription
                                        terminator with the base
                                        sequence given in Figure 1

Host:    recA
         lacI$^q$
         Suitable for fermentation (W3110)

Protein sequence design

As explained above, the protein comprises alternating crystalline and amorphous regions.

The crystalline unit repeat, C has the following amino acid sequence:

(GAGAGSGAAG(GAGAGS)$_8$Y)

The amorphous repeat unit, A1, based on a fragment of the lacz gene has the following sequence of 34 amino acids:

(KNSLAVVLQRRDWENPGVTQLNRLAAHPPFASTM)

The final amorphous unit, A2, at one end of the protein, has the following amino acid sequence:

(VLSAGRYHYQLVWCQK)

A2 is the amino acid sequence at the end of the lac Z gene, most of which is removed by a Pvu II cleavage, as will be described in the methodology following. Thus A2 is the final fragment of lac Z remaining after Pvu II cleavage.

Thus the design of the protein may be represented as:

met(C)$_n$A1(C)$_n$A1(C)$_n$A1(C)$_n$A1A2 (TerTerTer)

If the number of crystalline repeats is 4 (n = 4), the protein consists of: 59 59 59 59 34 59 59 59 59 34 59 59 59 59 34 59 59 59 59 34 16
met C C C C A1 C C C C A1 C C C C A1 C C C C A1 A2 (TerTerTer)

The total size of the protein is 1101 amino acids, and is encoded by a 3.3kb gene.

## DNA sequence design

The DNA sequence is designed with the following aims:
1. To minimise GC content (the gly and ala codons are GC rich);
2. To minimise secondary structures (to prevent inhibition of translation);
3. To optimise codon usage (for optimal expression).

DNA sequences were also designed for ease of in vitro manipulation with useful restriction endonuclease sites for:
a) polymerisation of sequences;
b) flexibility of design of structural variants.

## Gene assembly for poly (59 mer)

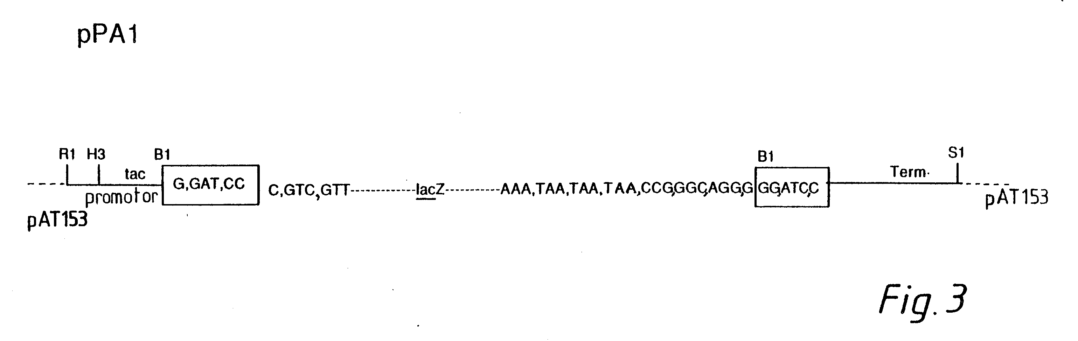

The basic unit of the 'silk' gene i.e.(C)₄A1 is represented by the Nco I fragment of the plasmid pPA10 (see Figure 2). Digestion of pPA10 by Nco I liberates the 810 bp 'silk' fragment that can be oligomerised to give higher homologues of the crystalline/amorphous repeat e.g. ((C)₄A1)₄

The starting point in the assembly of pPA10 was the pAT153-derived plasmid pPA1 (see Figure 3).

pPA1 is a pAT153 derivative carrying the tac promotor on a Hind III - Bam HI fragment and a synthetic terminator on a Bam HI - Sal I fragment. The promotor / terminator fragment (Hind III - Sal I) replaces the Hind III - Sal I region of pAT153 with concomitant loss of tetracycline resistance. It carries a 3.3kb lacZ gene cloned into the unique BamH I site. The lacZ gene is on a BamH I fragment derived from the beta-galactosidase gene fusion vector pMC1871 (Pharmacia). The lacZ gene lacks an initiator codon in this construct and should therefore be phenotypically Lac⁻.

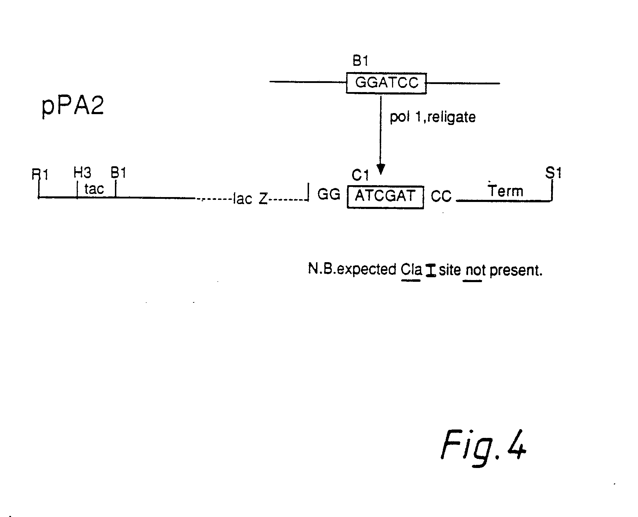

pPA2 was then constructed from pPA1 by partial digestion with BamHI, Pol I filling and re-ligation (Figure 4). This procedure destroys the BamH I site and should replace it with a Cla I site.

pPA2 is an isolate from the above procedure that was found to have lost the downstream BamH I site but retained the upstream site. Subsequent analysis revealed that the extra Cla I site had not been created (presumably due to nibbling), but since the site is downstream of the stop codon for lacZ it should not materially affect the constructions.

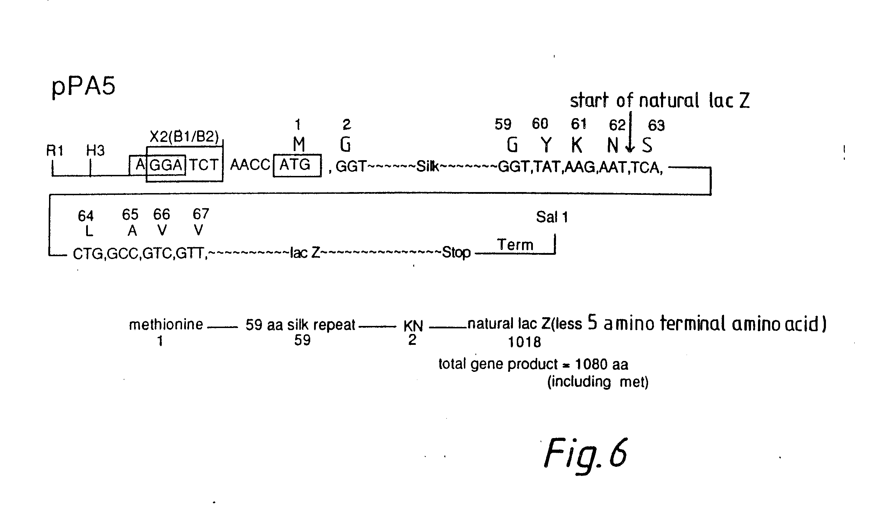

A 198 bp crystalline 'silk' gene was synthesised (Figure 5) and cloned into M13 mp19. pPA5 (Figure 6) was then constructed by introducing the 198bp gene into pPA2 on a Bgl II - Mst II fragment. This gave rise to silk-Bgal fusion (C lac Z) with an initiation codon provided by the silk fragment.

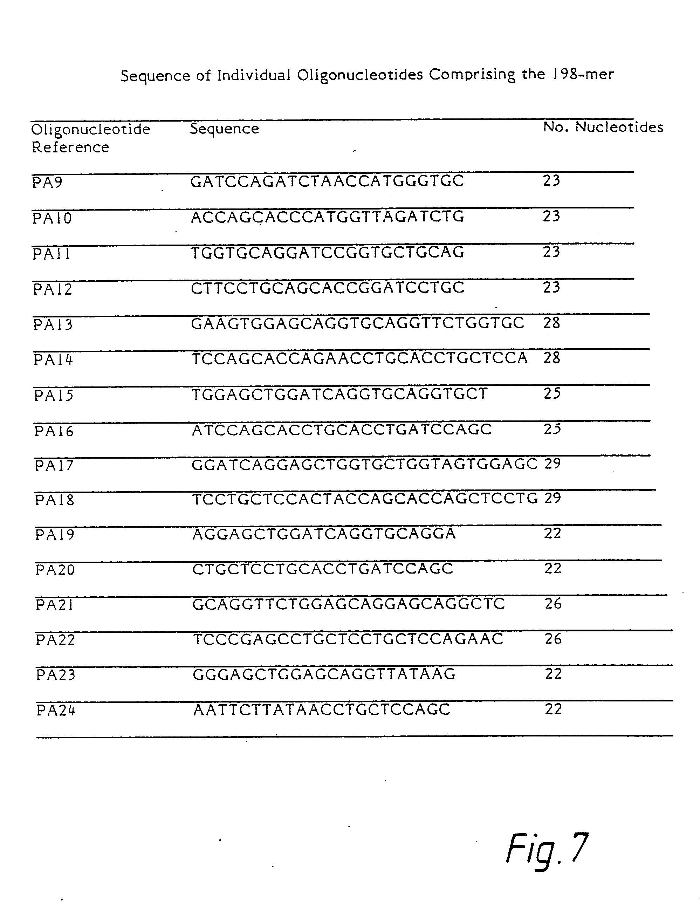

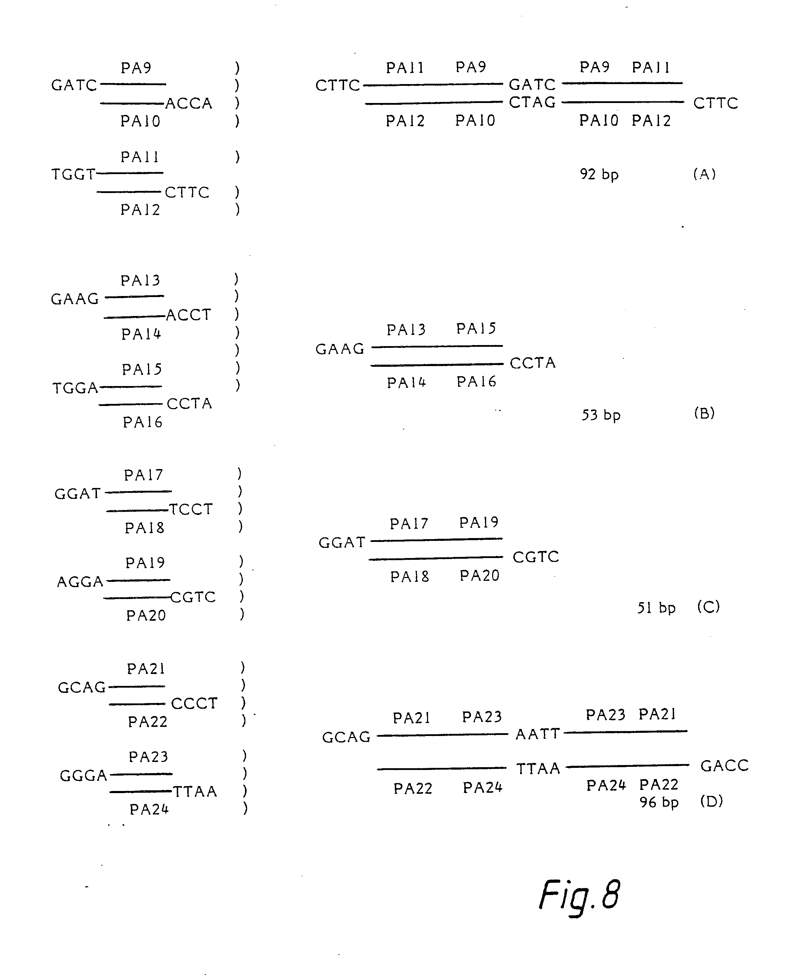

The 198-mer was constructed by synthesising 16 smaller oligonucleotides (PA9-PA24) of between 22 and 29 bases in length, as shown in Figures 5 and 7. Synthesis was carried out manually using beta-cyanoethylphosphoramidites and the oligonucleotides were purified using polyacrylamide gel electrophoresis. Each oligonucleotide was then phosphorylated at the 5' end and equimolar quantities of complementary pairs (e.g. PA9 and PA10) were annealed by heating to 90°C, followed by slow cooling. Adjacent double stranded oligonucleotides were then combined, annealed from 50°C and then ligated. This resulted in the production of four double stranded oligonucleotides, A, B, C and D, as shown in Figure 8.

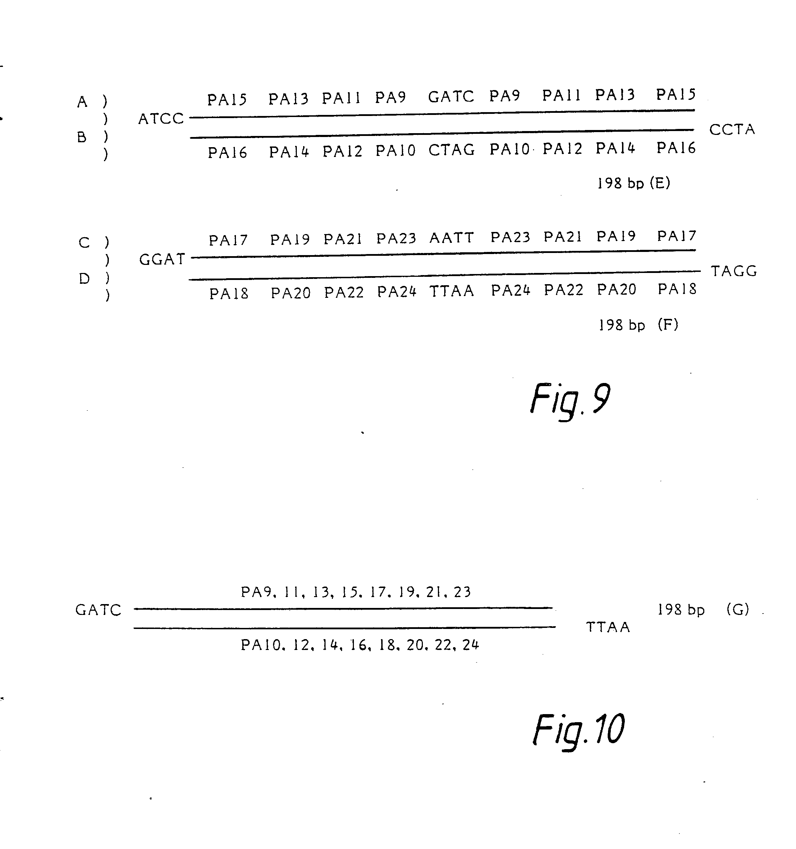

Oligonucleotides A and B shown in Figure 8 were then annealed and ligated: the resulting product was designated E. Oligonucleotides C and D shown in Figure 8 were treated similarly, resulting in the production of oligonucleotide F. E and F are shown in Figure 9.

Finally, equimolar quantities of E and F were combined, annealed and ligated.

The completed oligonucleotide was then digested back with Eco RI and Bam HI to give the desired overhanging ends on the 198-mer. The resulting product G is shown in Figure 10. (See also Figure 5).

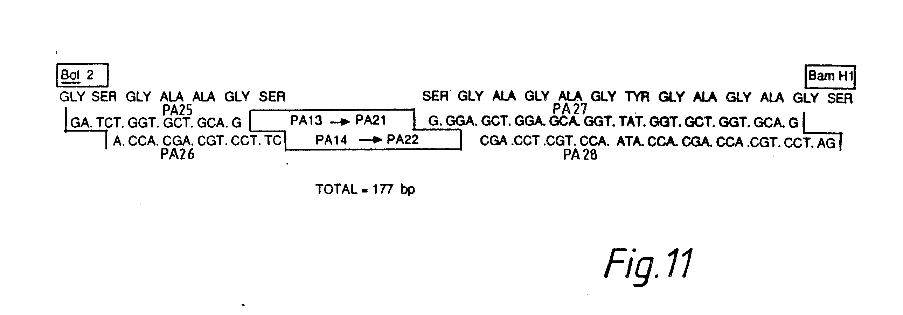

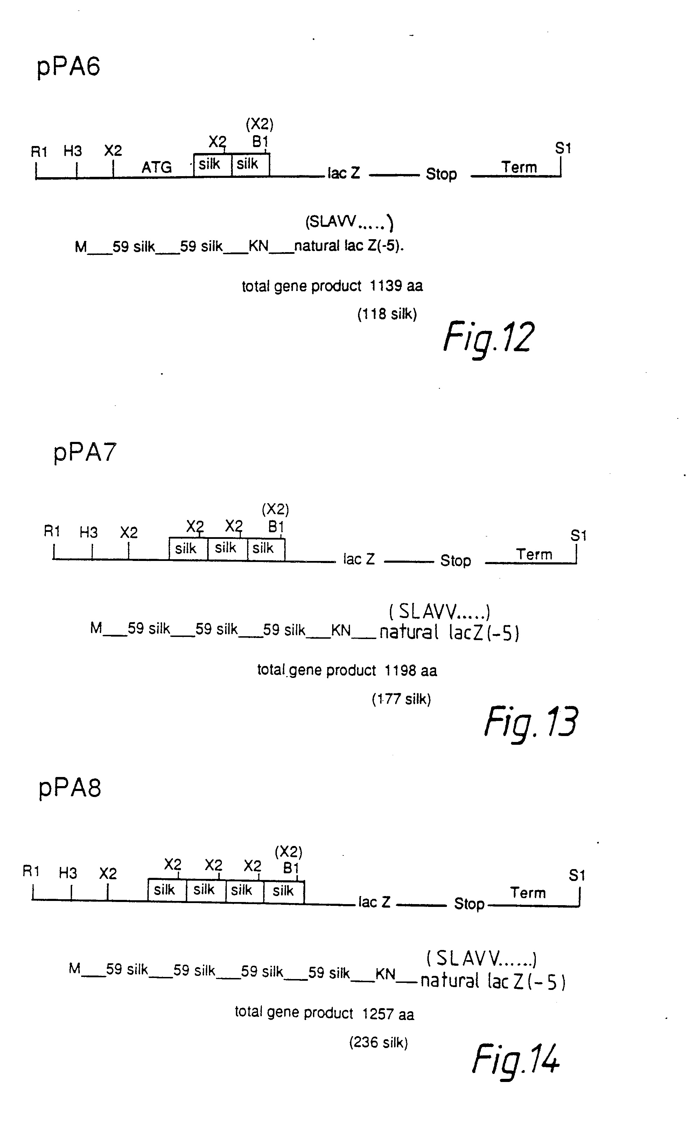

A 177 bp extension of 'crystalline silk' gene (Figure 11) was then cloned into the unique BamH I site of CIP-treated pPA5 to form pPA6 ((C)₂lac Z) (Figure 12). This 177 bp fragment was originally cloned into the BamHI site of M13mp19 and the gene excised on an Xho II fragment. This fragment carries one BamH I and one Bgl I end. Synthesis of the 177-mer was carried out in a similar fashion to the synthesis of the 198-mer.

As shown in Figure 11, oligonucleotides PA13 - PA22 were used as the core of the 177-mer, this central portion being surronded by PA25 and PA26 at one end and PA27 and PA28 at the other. Because the 177-mer has complementary overhanging 5' ends, the oligonucleotides containing these ends (i.e. PA25 and PA28) did not undergo initial 5' phosphorylation, in order to prevent circularization of the oligonucleotide when cloned into pPA5.

Note that cloning the 177-mer into pPA5 gives rise to only one BamH I site plus one BamHI/BglII junction that is refractory to cleavage by BamH I or Bgl II but is cut by Xho II.

The unique BamHI site in pPA6 can then be used to clone a further copy of the 177-mer to give pPA7 ((C)$_3$lac Z), again regenerating a unique BamH I site (Figure 13).

A further repetition of this cycle gave rise to pPA8 ((C)$_4$lac Z) in which the original '198 bp' silk fragment is joined to three 177 bp fragments (Figure 14).

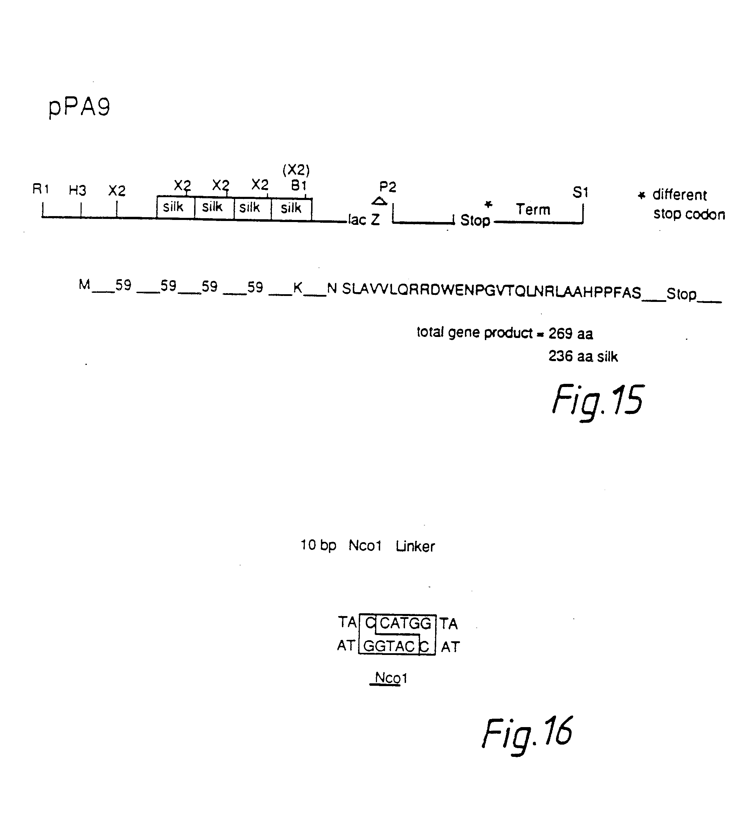

pPA9 (Figure 15) is a derivative of pPA8 which carries a deletion of most of lac Z: this was made by digestion with Pvu II and religation. This removed the two Pvu II fragments internal to lac Z and yielded an 880 bp 'silk' gene ((C)$_4$A1). The C-terminus of lacZ is frame shifted but termination occurs at an alternative stop codon close to the natural end of the gene.

pPA10 (Figure 2) is similar to pPA9 in that it carries a Pvu II deletion of pPA8 except that a 10 bp Nco I linker (Figure 16) was incorporated at the site of the deletion. The silk gene now terminates at the natural lacz stop. Digestion of pPA10 by Nco I liberates an 810 bp fragment that can be oligomerised to give higher homologues of the crystalline/amorphous repeat.

## Opportunites in modification of gene/protein structure

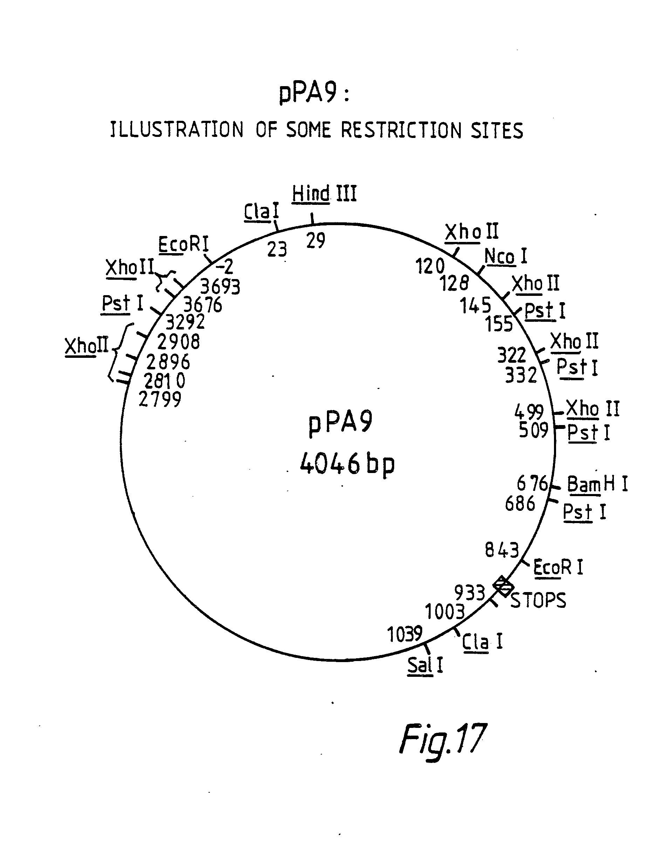

The restriction sites in pPA9 for example (see Figure 17) show the flexibility that is available for modifying the gene/protein structure by adding or deleting fractions at those sites.

An example of an interesting set of modifications is changing the length and amino acid sequence of the amorphous regions to alter protein behaviour.

The various plasmids can be transformed in conventional manner into a suitable bacterial host, for example E. Coli, for expression of "silk protein".

## Claims

1. A recombinant plasmid containing a DNA unit coding for the amino acid sequence (C):
(GAGAGSGAAG(GAGAGS)$_8$Y)

2. A plasmid according to claim 1, comprising several DNA units coding for said amino acid sequence, with the DNA units arranged in 2 to 6 groups each comprising 4 to 8 contiguous units, the groups being separated by smaller amorphous DNA regions coding for amino acid sequences of amorphous structure.

3. A plasmid according to claim 2, comprising an amorphous region (A1) based on part of the lac Z gene and coding for the following amino acid sequence:
(KNSLAVVLQRRDWENPGVTQLNRLAAHPPFASTM)

4. A plasmid according to claim 2 or 3, wherein the amino acid sequence includes a different amorphous group A2 at one end, and A2 is the base sequence at the end of lacZ gene remaining after PvuII cleavage and which codes for the following amino acid sequence:
(VSLAGRYHYQLVWCQK)

5. A plasmid according to claim 4, comprising DNA coding for protein of the following form:
met(C)$_n$A1(C)$_n$A1(C)$_n$A1(C)$_n$A1A2 (TerTerTer).

6. DNA coding for the amino acid sequence (C):
(GAGAGSGAAG(GAGAGS)$_8$Y)

7. DNA consisting of the base sequence shown in Figure 5.

8. A recombinant plasmid containing DNA consisting of the basic sequence shown in Figure 5.

9. A microorganism into which a plasmid in accordance with any one of claims 1 to 5 or 8 has been introduced.

10. A method of producing protein including the amino acid sequence (C):
(GAGAGSGAAG(GAGAGS)$_8$Y)
comprising inserting a plasmid in accordance with any one of claims 1 to 5 or 8 into a suitable microbial host in which the protein is expressed.

```
GG  ATCC CGGGCGATTCTACGCCCGGGTTTTTTAT GTCGAC CGATGCCC
 BamHI                                Sal I
```

## Fig.1

pPA10

NB. 810 bp <u>Nco I</u>  fragment can be polymerised to give larger silk genes.

* reading usual stop sequence for lacZ

FIG 2

pPA1

Fig.3

pPA2

B1
GGATCC

pol 1,religate

R1   H3   B1
tac

--------lac Z-------- GG  ATCGAT  CC ____Term____  S1

C1

N.B. expected Cla I site not present.

*Fig.4*

198-MER SEQUENCE

```
      PA9                      PA11                         PA13
GATCCAGATCTAACCATGGGTGC|TGGTGCAGGATCCGGTGCTGCAG|GAAGTGGAGCAGGTGCAGGTTCTGGTGC|
    GTCTAGATTGGTACCCACGACCA|CGTCCTAGGCCACGACGTCCTTC|ACCTCGTCCACGTCCAAGACCACGACCT|
      PA10                     PA12                         PA14

      PA15                     PA17                         PA19
TGGAGCTGGATCAGGTGCAGGTGCT|GGATCAGGAGCTGGTGCTGGTAGTGGAGC|AGGAGCTGGATCAGGTGCAGGA|
    CGACCTAGTCCACGTCCACGACCTA|GTCCTCGACCACGACCATCACCTCGTCCT|CGACCTAGTCCACGTCCTCGTC|
      PA16                     PA18                         PA20

      PA21                     PA23
GCAGGTTCTGGAGCAGGAGCAGGCTC|GGGAGCTGGAGCAGGTTATAAG
    CAAGACCTCGTCCTCGTCCGAGCCCT|CGACCTCGTCCAATATTCTTAA
      PA22                     PA24
```

*Fig.5*

EP 0 294 979 A1

pPA5

start of natural lac Z

|   |   | X2(B1/B2) |   | 1 | 2 |   |   |   | 59 | 60 | 61 | 62 | 63 |
|---|---|-----------|---|---|---|---|---|---|----|----|----|----|----|
|   |   |           |   | M | G |   |   |   | G  | Y  | K  | N↓ | S  |

R1  H3    A GGA TCT   AACC ATG , GGT~~~~~~Silk~~~~~~~GGT,TAT,AAG,AAT,TCA, ——

| 64 | 65 | 66 | 67 |            | Sal 1 |
|----|----|----|----|            |-------|
| L  | A  | V  | V  |            |       |

└— CTG,GCC,GTC,GTT,~~~~~~~~~~lac Z~~~~~~~~~~~~~~~Stop —— Term ——┘

methionine —— 59 aa silk repeat —— KN ——natural lac Z(less 5 amino terminal amino acid)
1                    59              2              1018

total gene product ≈ 1080 aa
(including met)

*Fig.6*

EP 0 294 979 A1

Sequence of Individual Oligonucleotides Comprising the 198-mer

| Oligonucleotide Reference | Sequence | No. Nucleotides |
|---|---|---|
| PA9 | GATCCAGATCTAACCATGGGTGC | 23 |
| PA10 | ACCAGCACCCATGGTTAGATCTG | 23 |
| PA11 | TGGTGCAGGATCCGGTGCTGCAG | 23 |
| PA12 | CTTCCTGCAGCACCGGATCCTGC | 23 |
| PA13 | GAAGTGGAGCAGGTGCAGGTTCTGGTGC | 28 |
| PA14 | TCCAGCACCAGAACCTGCACCTGCTCCA | 28 |
| PA15 | TGGAGCTGGATCAGGTGCAGGTGCT | 25 |
| PA16 | ATCCAGCACCTGCACCTGATCCAGC | 25 |
| PA17 | GGATCAGGAGCTGGTGCTGGTAGTGGAGC | 29 |
| PA18 | TCCTGCTCCACTACCAGCACCAGCTCCTG | 29 |
| PA19 | AGGAGCTGGATCAGGTGCAGGA | 22 |
| PA20 | CTGCTCCTGCACCTGATCCAGC | 22 |
| PA21 | GCAGGTTCTGGAGCAGGAGCAGGCTC | 26 |
| PA22 | TCCCGAGCCTGCTCCTGCTCCAGAAC | 26 |
| PA23 | GGGAGCTGGAGCAGGTTATAAG | 22 |
| PA24 | AATTCTTATAACCTGCTCCAGC | 22 |

*Fig. 7*

*Fig.8*

Fig.9

Fig.10

EP 0 294 979 A1

| Bgl 2 | | | | | | | Bam H1 |
|---|---|---|---|---|---|---|---|

GLY SER GLY ALA ALA GLY SER    SER GLY ALA GLY ALA GLY TYR GLY ALA GLY ALA GLY SER

PA25

GA. TCT. GGT. GCT. GCA. G | PA13 → PA21 | G. GGA. GCT. GGA. GCA. GGT. TAT. GGT. GCT. GGT. GCA. G

PA27

A. CCA. CGA. CGT. CCT. TC | PA14 → PA22 | CGA .CCT .CGT. CCA. ATA. CCA. CGA. CCA .CGT. CCT. AG

PA26               PA28

TOTAL = 177 bp

*Fig.11*

## pPA6

R1  H3  X2     ATG  [silk | silk]  — lac Z —— Stop  [Term] S1
                     X2  (X2) B1

(SLAVV.....)

M___59 silk___59 silk___KN___natural lac Z(-5).

total gene product 1139 aa

(118 silk)

*Fig.12*

## pPA7

R1  H3  X2  [silk | silk | silk] — lac Z —— Stop  [Term] S1
            X2   X2  (X2) B1

( SLAVV.....)

M___59 silk___59 silk___59 silk___KN___natural lacZ(-5)

total gene product 1198 aa

(177 silk)

*Fig.13*

## pPA8

R1  H3  X2  [silk | silk | silk | silk] — lac Z —— Stop  [Term] S1
            X2   X2   X2  (X2) B1

( SLAVV......)

M___59 silk___59 silk___59 silk___59 silk___KN___natural lac Z(-5)

total gene product 1257 aa

(236 silk)

*Fig.14*

pPA9

total gene product = 269 aa

236 aa silk

*Fig.15*

10 bp  Nco1  Linker

Nco1

*Fig.16*

pPA9 :

ILLUSTRATION OF SOME RESTRICTION SITES

Fig.17

## DOCUMENTS CONSIDERED TO BE RELEVANT

EP 88304906.6

| Category | Citation of document with indication, where appropriate, of relevant passages | Relevant to claim | CLASSIFICATION OF THE APPLICATION (Int. Cl.4) |
|---|---|---|---|
| X | GB - A - 2 162 190 (PA CONSULTING SERVICES LIMITED)  * Claims 1,6,7,10,13,14,16 * | 1,2,5, 6,9,10 | C 12 N 15/00 C 07 H 21/04 C 12 N 1/20 C 12 P 21/00 |
| A | CHEMICAL ABSTRACTS, vol. 88, no. 23, June 5, 1978, Columbus, Ohio, USA  J.F.MORROW et al. "Bacterial plasmids containing silk gene sequences" page 294, right column, abstract-no. 166 571d  & Miami Winter Symp. 1977, 13 (Mol.Cloning Recomb. DNA), 161-71 | 1,6 | |
| A | CHEMICAL ABSTRACTS, vol. 88, no. 1, January 2, 1978, Columbus, Ohio, USA  J.F.MORROW et al. "Studies on the silk fibroin gene" page 320, right column, abstract-no. 3 473j  & Miles Int. Symp. Ser. 1977, 10 (Recomb. Mol.iImpact Sci.Soc.), 409-17 | 1,6 | TECHNICAL FIELDS SEARCHED (Int. Cl.4) C 12 N C 12 P |

The present search report has been drawn up for all claims

| Place of search | Date of completion of the search | Examiner |
|---|---|---|
| VIENNA | 15-09-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

| | **DOCUMENTS CONSIDERED TO BE RELEVANT** | | EP 88304906.6 | |
|---|---|---|---|---|

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 88, no. 23, June 5, 1978, Columbus, Ohio, USA<br><br>R.F.MANNING et al. "Physical map of the Bombyx mori DNA containing the gene for silk fibroin"<br>page 339, left column, abstract-no. 167 078k<br><br>& J.Biol.Chem. 1978, 253 (6), 2044-52<br><br>-- | 1,6 | |
| A | CHEMICAL ABSTRACTS, vol. 103, no. 23, December 9, 1985, Columbus, Ohio, USA<br><br>K.KIMURA et al. "Molecular cloning of the fibroin light chain com-plementary DNA and its use in the study of the expression of the light chain gene in the posterior silk gland of Bombyx mori"<br>page 190, left, right column, abstract-no. 190 885h<br><br>& Experientia 1985, 41 (9), 1167-71<br><br>---- | 1,6 | **TECHNICAL FIELDS SEARCHED (Int. Cl.4)** |

The present search report has been drawn up for all claims

| Place of search | Date of completion of the search | Examiner |
|---|---|---|
| VIENNA | 15-09-1988 | WOLF |